# EUROPÄISCHE PATENTSCHRIFT

(11) **EP 4 045 834 B1**
(45) Veröffentlichungstag und Bekanntmachung des Hinweises auf die Patenterteilung: **21.06.2023**
(21) Anmeldenummer: 20793597.4
(22) Anmeldetag: 14.10.2020
(51) Int. Cl.: F16L 41/00, F01N 13/00, G01N 27/407

(54) **GEWINDESTUTZEN UND ABGASLEITUNG MIT GEWINDESTUTZEN UND ABGASSENSOR**
THREADED COUPLING, AND EXHAUST GAS LINE WITH THREADED COUPLING AND EXHAUST GAS SENSOR
RACCORD FILETÉ ET CONDUITE DE GAZ D'ÉCHAPPEMENT AVEC RACCORD FILETÉ ET CAPTEUR DE GAZ D'ÉCHAPPEMENT

(30) Priorität: 14.10.2019 DE 102019215786
(43) Veröffentlichungstag der Anmeldung: 24.08.2022
(73) Patentinhaber: Robert Bosch GmbH, 70442 Stuttgart (DE)
(72) Erfinder: KLOOS, Manuel, 70176 Stuttgart (DE); FUCHS, Timo, 71636 Ludwigsburg (DE); RUSS, Sebastian, 71277 Rutesheim (DE)
(86) Internationale Anmeldenummer: PCT/EP2020/078864
(87) Internationale Veröffentlichungsnummer: WO 2021/074205

(56) Entgegenhaltungen:
- EP-A2- 2 423 556
- DE-A1- 19 941 188
- DE-A1-102013 222 594
- DE-A1-102014 212 862

## Beschreibung

Die Erfindung betrifft das technische Gebiet der Gewindestutzen.

### Stand der Technik

Aus dem Stand der Technik, z.B. DE 10 2012 205 618 A1 oder aus EP 2 971 631 B1, sind bereits Gewindestutzen zum Einschweißen in die Abgasleitung einer Brennkraftmaschine und zur Aufnahme eines Abgassensors mit einer Durchgangsbohrung bekannt. In der Durchgangsbohrung ist eine Dichtfläche zur dichtenden Anlage des Abgassensors vorgesehen.

Es besteht weiterhin der Wunsch, die Dichtigkeit des Gewindestutzens bei eingeschraubtem Abgassensor nachhaltig zu verbessern bzw. über Lebensdauer zu gewährleisten.

### Offenbarung der Erfindung

Die Erfindung basiert zunächst auf den von den Erfindern identifizierten konkreten Anforderungen zur Gewährleistung der Dichtigkeit des Gewindestutzens. So wurde zum einen die Anforderung identifiziert, die Dichtfläche mit einer gewissen Flexibilität zu versehen, damit Unebenheiten der Dichtfläche, wie sie aus dem Einschweißen des (in der Regel planen) Gewindestutzens in die (in der Regel rohrförmige) Abgasleitung resultieren können, ausgleichbar sind. Dies gewährleistet eine gute Dichtwirkung zwischen dem Gewindestutzen und dem Abgassensor. Anderseits wurde aber auch die Anforderung identifiziert, die Vorrichtung mit einer möglichst großen Robustheit auszulegen, beispielsweise einer großen Dichtfläche, die einen guten Schutz gegen korrosive Unterwanderung gewährleistet.

Erfindungsgemäß werden beide Anforderungen zugleich dadurch realisiert, dass in der Durchgangsbohrung eine umlaufende, radial einwärts weisende Nase zur Anlage des Abgassensors vorgesehen ist, die von dem dem Abgas zugewandten Ende der Durchgangsbohrung beabstandet ist und von dem dem Abgas abgewandten Ende der Durchgangsbohrung beabstandet ist. Diese Anordnung der Nase "im Inneren" der Bohrung, also von ihren beiden Enden beabstandet, gewährleistet eine günstige mechanische Einleitung der auf die Dichtfläche einwirkenden Spannungen in das Innere des Gewindestutzens.

Unter einer Nase wird im Rahmen der Anmeldung insbesondere grundsätzlich eine Gestalt verstanden, mit einer Oberseite und einer Unterseite, die an einer Kante zusammenlaufen oder durch zumindest eine weitere Fläche miteinander verbunden sind.

Bei einer radial umlaufenden Nase sind die Oberseite und die Unterseite insbesondere jeweils in der Gestalt einer Ringfläche ausgebildet, beispielsweise einer ebenen oder konischen Ringfläche mit beispielsweise konstanter Erstreckung in die radiale Richtung (Breite).

Es kann vorgesehen sein, dass auf der dem Abgas zugewandten Seite der Nase ein Innengewinde in der Durchgangsbohrung vorgesehen ist. Der Abgassensor kann dann über ein komplementäres Außengewinde in das Innengewinde einschraubbar sein, bis er an der Nase entlang einer Dichtlinie oder auf einer Dichtfläche zur dichtenden Anlage kommt.

Es kann vorgesehen sein, dass die in die vom Abgas weg weisende Fläche der Nase räumlich plan und senkrecht zur axialen Richtung ausgebildet ist. In diesem Fall kann eine ebenfalls plane Fläche des Abgassensors zur dichtenden Anlage an der vom Abgas weg weisenden Fläche des Gewindestutzens vorgesehen sein. Die planen Flächen sind einerseits leicht zu fertigen und gewährleisten andererseits eine hohe Robustheit der Dichtung.

Es kann vorgesehen sein, dass die in die abgasseitige Richtung weisende Fläche der Nase und die die vom Abgas weg weisende Fläche der Nase beide plan und zueinander parallel sind, insbesondere beide senkrecht zur axialen Richtung sind. Solche Nasen lassen sich leichtfertigen.

Es kann alternativ vorgesehen sein, dass die zum Abgas hin weisende Fläche der Nase konisch ausgebildet ist, wobei die Normalen auf dieser konischen Fläche einen Winkel mit der axialen Richtung einschließen, der zwischen 0° und 25°, insbesondere zwischen 1° und 25°, liegt. Es hat sich nämlich gezeigt, dass bei solchen Nasen die Einleitung der Materialspannungen aus der Nase in den Gewindestutzen mit weniger lokalen Spannungsspitzen erfolgt als bei Nasen mit planen und zueinander parallelen Ober- und Unterseiten. Die Robustheit des Gewindestutzens lässt sich also auf diese Weise erhöhen, insbesondere wenn vorgesehen ist, dass sich die Nase nach radial innen kontinuierlich verjüngt.

Dimensionierungen, die sich in Untersuchungen der Anmelderin bewährt haben, sehen vor, dass die Erstreckung der Nase in die axiale Richtung an ihrem radial innerem Ende 0,5mm bis 3mm beträgt. An dieser Stelle kann sich beispielsweise eine Ringfläche befinden, die parallel zur axialen Richtung ausgerichtet ist.

Eine hohe Stabilität des Gewindestutzens ist insbesondere gewährleistet, wenn die Nase von dem dem Abgas zugewandten Ende der Durchgangsbohrung in axialer Richtung um das Maß d beabstandet ist, wobei d nicht kleiner ist als die Erstreckung der Nase in die axiale Richtung an ihrem radial innerem Ende, 5% des engsten Durchmessers der Durchgangsbohrung und/oder 7% der Erstreckung des Gewindestutzens in axialer Richtung.

Die Erfindung betrifft auch einen solchen Gewindestutzen, wobei in ihn ein Abgassensor so eingeschraubt ist, dass eine Anlagefläche des Abgassensors an der Nase des Gewindestutzens zur dichtenden Anlage kommt; ferner eine Abgasleitung, in die ein solcher Gewindestutzen (mit oder ohne eingeschraubten Abgassensor) eingeschweißt ist.

Derartige Gewindestutzen können in einfacher Weise hergestellt werden, indem die Durchgangsbohrung durch eine erste Bohrung in die axiale Richtung und eine zweite Bohrung in Gegenrichtung eingebracht wird, wobei axial zwischen der ersten Bohrung und der zweiten Bohrung die Nase verbleibt.

An die Stelle des Abgassensors kann auch eine andere in den Gewindestutzen einschraubbare Vorrichtung treten, beispielsweise ein Dosiermodul oder eine Einspritzdüse. Es kann sich ggf. sogar um einen Blindstopfen handeln.

### Ausführungsbeispiele

Die Erfindung wird im Folgenden anhand der Figuren näher erläutert.
Figur 1 zeigt eine Abgasleitung, in die ein Gewindestutzen eingeschweißt ist, in den ein Abgassensor eingeschraubt ist, gemäß dem Stand der Technik.
Figur 2 zeigt einen erfindungsgemäß modifizierten Gewindestutzen, in den ein Abgassensor eingeschraubt ist (Abgassensor ist nur teilweise dargestellt).
Figur 3 zeigt Teilansichten erfindungsgemäßer Gewindestutzen, in denen insbesondere die Nasen dargestellt sind. Anhand der Schraffuren sind die mechanischen Spannungsverläufe in der Vorrichtung sichtbar gemacht.

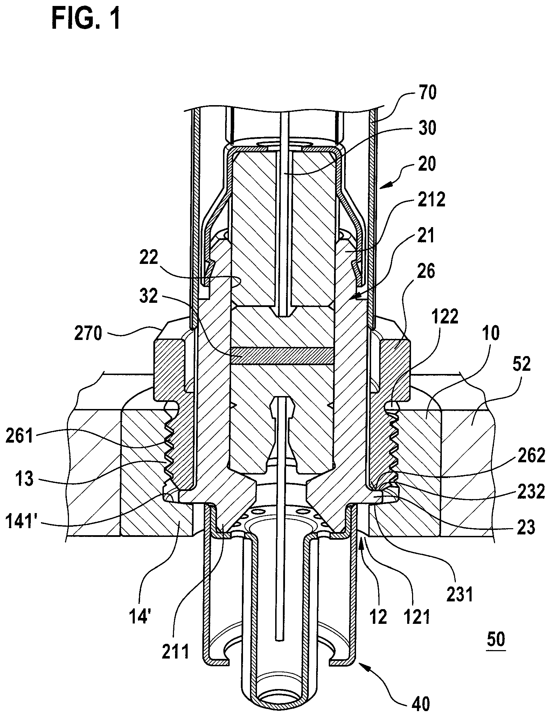

Die Figur 1 zeigt einen Gewindestutzen 10, der in eine Wand 52 einer Abgasleitung 50 (nachfolgend auch: "Abgas") einer nicht gezeichneten Brennkraftmaschine eigeschweißt ist. Der Gewindestutzen 10 weist eine Durchgangsbohrung 12 auf, die in axialer Richtung ein dem Abgas zugewandtes Ende 121 und ein dem Abgas abgewandtes Ende 122 aufweist.

Die Durchgangsbohrung 12 des dargestellten, aus dem Stand der Technik bekannten Gewindestutzens 10 weist ein Innengewinde 13 auf und ist an ihrem dem Abgas zugewandten Ende 121 durch einen nach innen weisenden Abschnitt 14' verengt. Der nach innen weisenden Abschnitt 14' weist eine vom Abgas weg weisende Ringschulter 141' auf, an der der Sensor 20 zur dichtenden Anlage kommt.

Der Sensor 20 weist ein etwa hülsenförmiges Gehäuse 21 auf. In einer Durchgangsbohrung 22 des Gehäuses 21 ist ein keramisches Sensorelement 30 durch eine keramische Dichtung 32 gehalten. Am abgasseitigen Ende 211 des Gehäuses 21 ist ein aus zwei Schutzrohren bestehendes Schutzrohrmodul 40 befestigt; an dem vom Abgas abgewandten Ende 212 des Gehäuses 21 ist eine Schutzhülse 70 befestigt.

Das Gehäuse 21 des Sensors 20 weist eine umlaufende, nach außen abragende Ringschulter 23 auf. Die zum Abgas weisende Fläche 231 dieser Ringschulter 23 liegt dichtend auf der vom Abgas weg weisenden Ringschulter 141' des Gewindestutzens 20 auf.

Der Abgassensor 20 weist ferner eine außen auf dem Gehäuse 21 verschiebbare Überwurfschraube 26 auf. Diese weist ein Außengewinde 261 auf, das mit dem Innengewinde 13 des Gewindestutzens kompatibel ist, und weist auf der dem Abgas abgewandten Seite des Außengewindes 261 ein Außensechskantprofil 270 auf. Auf diese Weise ist eine dem Abgas abgewandte Fläche 232 der Ringschulter 23 mit einer dem Abgas zugewandten Seite 262 der Überwurfschraube 26 beaufschlagbar, sodass die zum Abgas weisende Fläche 231 der Ringschulter 23 des Abgassensors 20 auf die vom Abgas weg weisenden Ringschulter 141' des Gewindestutzens 10 aufgepresst wird.

Die axiale Richtung verläuft von oben nach unten in der Figur 1 (sowie in den weiteren Figuren) und entspricht der Einschraubrichtung des Abgassensors 20 in den Gewindestutzen 10.

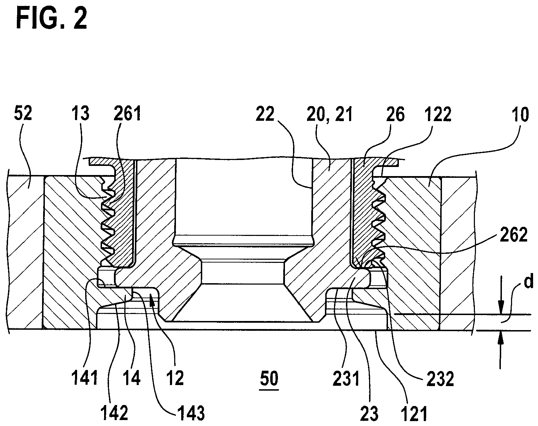

Die Figur 2 zeigt den erfindungsgemäß modifizieren Gewindestutzen 10 und ausschnittsweise den Abgassensor 20, der im Beispiel mit dem in Figur 1 gezeigten Abgassensor 20 übereinstimmen kann.

Der in der Figur 2 gezeigte erfindungsgemäße Gewindestutzen 10 unterscheidet sich von dem in der Figur 1 gezeigten, vorbekannten Gewindestutzen dadurch, dass in der Durchgangsbohrung 12 eine umlaufende, radial einwärts weisende Nase 14 zur Anlage des Abgassensors vorgesehen ist, die von dem dem Abgas zugewandten Ende 121 der Durchgangsbohrung 12 beabstandet ist und von dem dem Abgas abgewandten Ende 122 der Durchgangsbohrung 12 beabstandet ist.

Die Nase 14 ist eine Ausgestaltung mit einer Oberseite 141, die im Beispiel eine ebene Ringfläche konstanter Breite ist, die von dem Abgas weg weist, und mit einer im Beispiel konischen Unterseite 142, die eine Ringfläche konstanter Breite ist und zum Abgas hin weist. Die Oberseite 141 und die Unterseite 142 der Nase sind im Beispiel durch eine Innenfläche 143, die sich parallel zur axialen Richtung erstreckt, verbunden. Im Beispiel ist die Normale auf der Unterseite 142 um 14° gegen die axiale Richtung verkippt, sodass sich die Nase 14 nach radial innen kontinuierlich verjüngt.

In diesem Beispiel beträgt die Erstreckung der Nase 14 in die axiale Richtung an ihrem radial innerem Ende (also die Erstreckung der Innenfläche 143 in diese Richtung) 0,5mm bis 3mm.

Die Nase 14 ist von dem dem Abgas zugewandten Ende 121 der Durchgangsbohrung 12 in axialer Richtung um das Maß d beabstandet, erfindungsgemäß ist d > 0mm.

Es kann vorgesehen sein, dass d nicht kleiner ist als die Erstreckung der Nase 14 in die axiale Richtung an ihrem radial innerem Ende, 5% des engsten Durchmessers der Durchgangsbohrung 12 und/oder 7% der Erstreckung des Gewindestutzens 10 in axialer Richtung

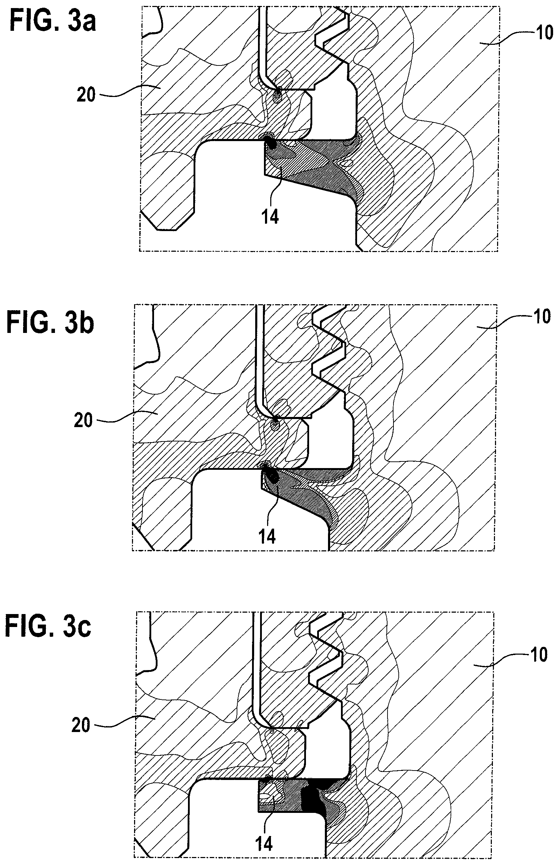

Die Figur 3 zeigt in den Teilfiguren a, b und c ausschnittsweise erfindungsgemäße Gewindestutzen 10. Die im Inneren des Gewindestutzens 10 wirkenden mechanischen Spannungen sind durch Schraffuren kenntlich gemacht sind, wobei dichte Schraffuren hohe mechanische Spannungen bedeuten.

Die in der Figur 2 gezeigte Vorrichtung entspricht der der Figur 3a. Bei der Figur 3b ist der Winkel zwischen Oberseite 141 und Unterseite 142 der Nase 14 auf 20° erhöht. In der Figur 3c sind die Oberseite 141 und die Unterseite 142 der Nase 14 beide eben und - senkrecht zur axialen Richtung - parallel zueinander.

Es wird ersichtlich, dass die erfindungsgemäße Anordnung der Nase 14 bewirkt, das mechanische Spannungen, die aus der Auflage des Sensors 20 auf der Nase 14 resultieren, in den abgasseitig der Nase 14 gelegenen Bereich des Gewindestutzens 10 eingeleitet werden können. Eine übermäßige Spannungsüberhöhung in dem Gewindestutzen 10 wird so vermindert. Es resultiert eine hohe Robustheit bei gleichzeitiger Flexibilität der Dichtfläche, sodass gewisse Formabweichungen zwischen Gewindestutzen 10 und Abgassensor 20 ausgleichbar werden.

Es ist ferner ersichtlich, dass diese Effekte bei der Ausgestaltung gemäß den Figuren 3a und 3b nochmals verstärkt sind und die Spannungen besser und homogener, also mit weniger Spannungsspitzen von der Nase 14 in den Gewindestutzen 10 eingeleitet werden. Bei diesen Ausgestaltungen ist vorgesehen, dass die zum Abgas hin weisende Fläche 142 der Nase 14 konisch ausgebildet ist, wobei die Normalen auf dieser konischen Fläche einen Winkel mit der axialen Richtung einschließen, der zwischen 0° und 25° liegt, sodass sich die Nase 14 nach radial innen kontinuierlich verjüngt.

## Patentansprüche

1. Gewindestutzen zum Einschweißen in die Abgasleitung (50) einer Brennkraftmaschine und zur Aufnahme eines Abgassensors (20), mit einer Durchgangsbohrung (12), die in axialer Richtung ein dem Abgas zugewandtes Ende (121) und ein dem Abgas abgewandtes Ende (122) aufweist, **dadurch gekennzeichnet, dass** in der Durchgangsbohrung (12) eine umlaufende, radial einwärts weisende Nase (14) zur Anlage des Abgassensors (20) vorgesehen ist, die von dem dem Abgas zugewandten Ende (121) beabstandet ist und von dem dem Abgas abgewandten Ende (122) beabstandet ist.

2. Gewindestutzen nach Anspruch 1, **dadurch gekennzeichnet, dass** auf der vom Abgas abgewandten Seite der Nase (14) ein Innengewinde (13) in der Durchgangsbohrung (12) vorgesehen ist.

3. Gewindestutzen nach Anspruch 1 oder 2, **dadurch gekennzeichnet, dass** die vom Abgas weg weisende Fläche (141) der Nase (14) räumlich plan und senkrecht zur axialen Richtung ausgebildet ist.

4. Gewindestutzen nach einem der vorangehenden Ansprüche, **dadurch gekennzeichnet, dass** die zum Abgas hin weisende Fläche (142) der Nase (14) konisch ausgebildet ist, wobei die Normalen auf der zum Abgas hin weisenden Fläche (142) einen Winkel mit der axialen Richtung einschließen, der zwischen 0° und 25° liegt.

5. Gewindestutzen nach einem der vorangehenden Ansprüche, **dadurch gekennzeichnet, dass** sich die Nase (14) nach radial innen kontinuierlich verjüngt.

6. Gewindestutzen nach einem der vorangehenden Ansprüchen, **dadurch gekennzeichnet, dass** die Erstreckung der Nase (14) in die axiale Richtung an ihrem radial innerem Ende 0,5mm bis 3mm beträgt.

7. Gewindestutzen nach einem der vorangehenden Ansprüche, **dadurch gekennzeichnet, dass** die Nase (14) von dem dem Abgas zugewandten Ende (121) der Durchgangsbohrung (12) in axialer Richtung um das Maß d beabstandet ist, wobei d nicht kleiner ist als die Erstreckung der Nase (14) in die axiale Richtung an ihrem radial innerem Ende, 5% des engsten Durchmessers der Durchgangsbohrung 12 und/oder 7% der Erstreckung des Gewindestutzens (10) in axialer Richtung.

8. Gewindestutzenvorrichtung aus einem Gewindestutzen (10) nach einem der vorangehenden Ansprüche und einem Abgassensor (20), **dadurch gekennzeichnet, dass** in den Gewindestutzen (10) der Abgassensor (20) so eingeschraubt so, dass eine Anlagefläche (232) des Abgassensors (20) an der Nase (14) des Gewindestutzens (10) zur dichtenden Anlage kommt.

9. Abgasleitung (50), in die ein Gewindestutzen (10) nach einem der Ansprüche 1 bis 7 oder der Gewindestutzen (10) einer Gewindestutzenvorrichtung nach Anspruch 8 eingeschweißt ist.

10. Gewindestutzen nach einem der Ansprüche 1 bis 7 oder Gewindestutzenvorrichtung nach Anspruch 8, **dadurch gekennzeichnet, dass** der Gewindestutzen (10) hergestellt wird, indem die Durchgangsbohrung (12) durch eine erste Bohrung in die axiale Richtung und eine zweite Bohrung in Gegenrichtung eingebracht wird, wobei axial zwischen der ersten Bohrung und der zweiten Bohrung die Nase (14) verbleibt.

## Claims

1. Threaded connecting piece for being welded into the exhaust-gas line (50) of an internal combustion engine and for receiving an exhaust-gas sensor (20), having a passage bore (12) which has, in an axial direction, an end (121) facing towards the exhaust gas and an end (122) facing away from the exhaust gas, **characterized in that**, in the passage bore (12), provision is made of an encircling, radially inwardly directed lug (14) for abutment of the exhaust-gas sensor (20), which lug is spaced apart from the end (121) facing towards the exhaust gas and is spaced apart from the end (122) facing away from the exhaust gas.

2. Threaded connecting piece according to Claim 1, **characterized in that**, on that side of the lug (14) which faces away from the exhaust gas, provision is made of an internal thread (13) in the passage bore (12) .

3. Threaded connecting piece according to Claim 1 or 2, **characterized in that** that surface (141) of the lug (14) which is directed away from the exhaust gas is configured to be spatially planar and perpendicular to the axial direction.

4. Threaded connecting piece according to one of the preceding claims, **characterized in that** that surface (142) of the lug (14) which is directed towards the exhaust gas is configured to be conical, wherein the normals to the surface (142) directed towards the exhaust gas include with the axial direction an angle that lies between 0° and 25°.

5. Threaded connecting piece according to one of the preceding claims, **characterized in that** the lug (14) narrows radially inwards continuously.

6. Threaded connecting piece according to one of the preceding claims, **characterized in that** the extent of the lug (14) in the axial direction at the radial inner end thereof is 0.5 mm to 3 mm.

7. Threaded connecting piece according to one of the preceding claims, **characterized in that** the lug (14) is spaced apart in the axial direction from that end (121) of the passage bore (12) which faces towards the exhaust gas by the extent d, wherein d is not less than the extent of the lug (14) in the axial direction at the radial inner end thereof, 5% of the narrowest diameter of passage bore (12) and/or 7% of the extent of the threaded connecting piece (10) in the axial direction.

8. Threaded-connecting-piece device composed of a threaded connecting piece (10) according to one of the preceding claims and an exhaust-gas sensor (20), **characterized in that** the exhaust-gas sensor (20) is screwed into the threaded connecting piece (10) in such a way that an abutment surface (232) of the exhaust-gas sensor (20) comes into sealing abutment against the lug (14) of the threaded connecting piece (10).

9. Exhaust-gas line (50) into which a threaded connecting piece (10) according to one of Claims 1 to 7 or the threaded connecting piece (10) of a threaded-connecting-piece device according to Claim 8 is welded.

10. Threaded connecting piece according to one of Claims 1 to 7 or threaded-connecting-piece device according to Claim 8, **characterized in that** the threaded connecting piece (10) is produced **in that** the passage bore (12) is formed by a first bore in the axial direction and a second bore in the opposite direction, wherein the lug (14) remains axially between the first bore and the second bore.

## Revendications

1. Raccord fileté destiné à être soudé dans la conduite de gaz d'échappement (50) d'un moteur à combustion interne et destiné à recevoir un capteur de gaz d'échappement (20), comportant un alésage traversant (12) qui présente, dans la direction axiale, une extrémité (121) tournée vers le gaz d'échappement et une extrémité (122) opposée au gaz d'échappement, **caractérisé en ce qu'**un ergot (14) périphérique, orienté radialement vers l'intérieur et servant à l'appui du capteur de gaz d'échappement (20) est prévu dans l'alésage traversant (12), lequel ergot est espacé de l'extrémité (121) tournée vers le gaz d'échappement et est espacé de l'extrémité (122) opposée au gaz d'échappement.

2. Raccord fileté selon la revendication 1, **caractérisé en ce qu'**un filetage intérieur (13) est prévu dans l'alésage traversant (12) sur le côté, opposé au gaz d'échappement, de l'ergot (14).

3. Raccord fileté selon la revendication 1 ou 2, **caractérisé en ce que** la surface (141) de l'ergot (14) qui est détournée du gaz d'échappement est réalisée de manière plane spatialement et perpendiculairement à la direction axiale.

4. Raccord fileté selon l'une des revendications précédentes, **caractérisé en ce que** la surface (142), tournée vers le gaz d'échappement, de l'ergot (14) est réalisée de manière conique, la normale à la surface (142) tournée vers le gaz d'échappement formant avec la direction axiale un angle qui est compris entre 0° et 25°.

5. Raccord fileté selon l'une des revendications précédentes, **caractérisé en ce que** l'ergot (14) se rétrécit de manière continue radialement vers l'intérieur.

6. Raccord fileté selon l'une des revendications précédentes, **caractérisé en ce que** l'étendue de l'ergot (14) dans la direction axiale à son extrémité radialement intérieure vaut de 0,5mm à 3mm.

7. Raccord fileté selon l'une des revendications précédentes, **caractérisé en ce que** l'ergot (14) est espacé, dans la direction axiale, de la mesure d, de l'extrémité (121) de l'alésage traversant (12) qui est tournée vers le gaz d'échappement, d n'étant pas inférieure à l'étendue de l'ergot (14) dans la direction axiale à son extrémité radialement intérieure, 5% du diamètre le plus étroit de l'alésage traversant (12) et/ou 7% de l'étendue du raccord fileté (10) dans la direction axiale.

8. Dispositif à raccord fileté constitué d'un raccord fileté (10) selon l'une des revendications précédentes et d'un capteur de gaz d'échappement (20), **caractérisé en ce que** le capteur de gaz d'échappement (20) est vissé dans le raccord fileté (10), de telle sorte qu'une surface d'appui (232) du capteur de gaz d'échappement (20) vienne en appui avec étanchéité contre l'ergot (14) du raccord fileté (10).

9. Conduite de gaz d'échappement (50), dans laquelle est soudé un raccord fileté (10) selon l'une des revendications 1 à 7 ou le raccord fileté (10) d'un dispositif à raccord fileté selon la revendication 8.

10. Raccord fileté selon l'une des revendications 1 à 7 ou dispositif à raccord fileté selon la revendication 8, **caractérisé en ce que** le raccord fileté (10) est produit par le fait que l'alésage traversant (12) est ménagé par un premier alésage dans la direction axiale et un deuxième alésage en sens inverse, l'ergot (14) demeurant axialement entre le premier alésage et le deuxième alésage.
